(19) Europäisches Patentamt
European Patent Office
Office européen des brevets

(11) Publication number: **0 033 181**
**B1**

(12) **EUROPEAN PATENT SPECIFICATION**

(45) Date of publication of patent specification: **23.11.83**

(21) Application number: **81200095.8**

(22) Date of filing: **27.01.81**

(51) Int. Cl.³: **C 07 C 87/30,**
C 07 C 93/04,
C 07 C 121/43,
C 07 C 87/06, C 07 C 87/14

# (54) Mixtures of branched-chain amines and derivatives thereof.

(30) Priority: **28.01.80 US 116116**

(43) Date of publication of application:
**05.08.81 Bulletin 81/31**

(45) Publication of the grant of the patent:
**23.11.83 Bulletin 83/47**

(84) Designated Contracting States:
**DE FR GB IT NL**

(56) References cited:
**EP - A - 0 008 839**
**GB - A - 1 067 519**
**GB - A - 1 226 571**
**GB - A - 1 296 352**

(73) Proprietor: **AKZO N.V.**
**Velperweg 76**
**NL-6824 BM Arnhem (NL)**

(72) Inventor: **McDaniel, Robert Stephen, Jr.**
**620 Rockhurst Rd.**
**Bolingbrook Illinois 60439 (US)**

(74) Representative: **Sieders, René et al,**
**P.O. Box 314**
**NL-6800 AH Arnhem (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Mixtures of branched-chain amines and derivatives thereof

This invention relates to a mixture of amines, at least part of which are branched-chain amines, or the secondary, tertiary or quaternary amines derived therefrom and containing 1 up to 3 N-substituted groups selected from the group consisting of substituted or unsubstituted alkyl groups having up to 40 carbon atoms, substituted or unsubstituted cycloalkyl groups having from 3 to 6 carbon atoms and/or polyalkylene oxide-containing groups having from 2 to 30 alkylene oxide units each containing from 2 to 4 carbon atoms. Branched-chain amines, especially aliphatic secondary amines are described in, inter alia, British Patent Specification 1 226 571. They correspond to the general formula $R_1CH_2NHCH_2R_2$, in which $R_1$ and $R_2$ represent branched-chain, aliphatic, saturated hydrocarbon radicals containing 15—24 carbon atoms.

These amines are said to be excellently suitable to be used as oxidation inhibitor in engine lubricating oils. Applicant has found, however, that for the last-mentioned use the known amines can be improved upon in several respects. This particularly applies to the pour point in the presence of straight chain amines.

The present invention provides a new class of amines having a surprisingly low pour point, even in the presence of certain impurities such as straight chain amines.

The present invention consists in that at least 10% by weight of the branched-chain amines conforms to the formula:

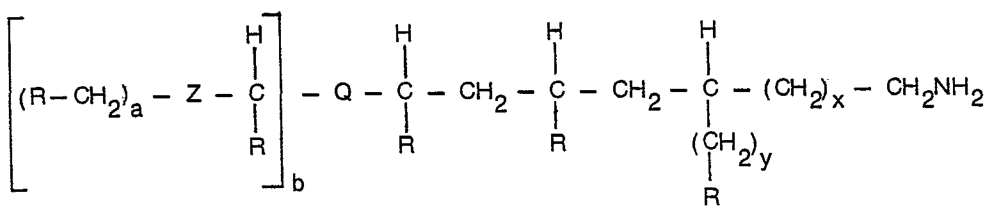

where x is 0 or 2 and where y is 2 or 0, and where
when y = 0, x = 2 and
when y = 2, x = 0;
$R = CH_3(CH_2)_n$, where n represents an integer of from 3 to 42 b = 0 or 1, where
where b = 0, Q represents a hydrogen atom, and
when b = 1, Q represents a $CH_2$-group, and
a = 0 or 1, where
when a = 0, Z represents a hydrogen atom, and
when a = 1, Z represents a $CH_2$-group.

It has been found that for a large number of uses of the compounds of the present invention it is preferred that they conform to the above formula where n is from about 3 to about 17. The compounds find application as corrosion inhibitors, in the mineral flotation, as fertilizer anticaking agents, as lubricant additives, fuel additives, mold release agents, fabric softeners, biocides, demulsification agents, in the preparation of surface active compounds and in the preparation of emulsifiers for bitumen.

Particularly with a view to the use in fabric softener compositions it is preferred to employ branched-chain amines of the above formula where n represents an integer of from 17 to 42.

For many applications the object will be to obtain good processing properties in combination with minimum volatility. According to the invention the secondary, tertiary or quaternary amines are made up then of at least two structural units of the above-formula.

Suitable compounds are obtained when the secondary, tertiary or quaternary amines are made up of 1 up to 3 substituted or unsubstituted alkyl groups having up to 22 carbon atoms, cycloalkyl groups having from 3 to 6 carbon atoms and/or polyalkylene oxide-containing groups having from 2 to 30 alkylene oxide units each containing 2 to 4 carbon atoms.

Suitable for use are a great many substitutes, such as lower alkyl or lower alkoxy groups, a halogen atom, a cyano group or a nitro group.

Highly valuable compounds are obtained if use is made of a hydroxyl group or an amino group. The amines of the above-formula can be obtained through reduction in a known manner from the corresponding nitrile. The nitrile is prepared from the corresponding carboxylic acid having the formula:

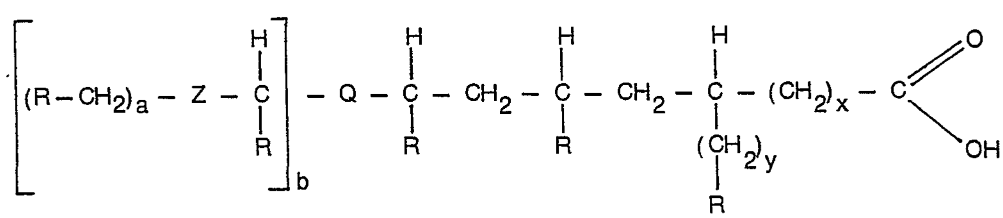

where R, Z, Q, a, b, x and y have the above indicated meanings.

The preparation of nitriles from carboxylic acids is well-known in the art (See e.g., Houben-Weyl, Methoden der organischen Chemie, 4 Ed., Bd. VIII, pp. 335—338).

According to a preferred process the carboxylic acid while in the liquid phase is converted into a mixture of carboxylic acid amide and nitrile, followed by a vapor phase step in which conversion into the nitrile is completed with the aid of a dehydration catalyst.

The primary amines are produced by hydrogenation using about 0.5 to 5%, preferably about 1 to about 2%, of a catalyst such as Raney nickel at hydrogen pressures of $10.10^5$—$140.10^5$ Pa and a temperature in the range of 110° to 200°C, or cobalt catalyst at hydrogen pressures of about $40.15^5$ to about $140.10^5$ Pa, preferably about $65.10^5$ to about $110.10^5$ Pa, and at a temperature from about 80°C to about 200°C, preferably about 145°C. Increased primary amine formation is favored by the use of alkaline conditions or ammonia plus a lower alcohol. Secondary amine formation is favored by higher temperatures (175°—250°C), exclusion of water, and continuous venting of ammonia.

Although both batch and continuous processes may be employed, it is believed preferable to utilize a continuous process.

Tertiary amines built up of one or two structural units according to the invention and two or one lower aliphatic radicals may be prepared by different methods.

Methyl groups may be introduced by the reaction between a primary or secondary amine having one or two branched-chain units according to the invention and formic acid and formaldehyde in accordance with the following reaction:

$$RNH_2 + 2\ CH_2O + 2\ HCOOH \rightarrow RN(CH_3)_2 + 2\ CO_2 + 2\ H_2O.$$

Mixed tertiary amines may be prepared by reductive alkylation of a branched-chain primary or secondary amine according to the invention. The amine is subjected to catalytic hydrogenation in the presence of an aldehyde, which may be represented by the following reaction:

$$RNH_2 + 2\ R'CHO + 2\ H_2 \rightarrow RN(CH_2R')_2 + 2\ H_2O.$$

The alkylene oxide derivatives of the present invention are prepared by the bringing a branched-chain primary amine according to the invention into reaction with a lower alkylene oxide such as ethylene or propylene oxide. The amino substituted secondary amines of the invention are prepared from a primary amine having the first-mentioned structural formula and acrylonitrile, followed by catalytic hydrogenation to the diamine:

$$RNH_2 + CH_2{=}CH-CN \rightarrow RNHCH_2CH_2CN \xrightarrow[(Ni)]{2H_2} RNHCH_2CH_2CH_2NH_2$$

The salts of the amines of the present invention may be derived both from organic and inorganic acids. Suitable inorganic acids include phosphoric acid, hydrochloric acid and sulphuric acid. Suitable organic acids are formic acid, acetic acid, propionic acid, butyric acid, stearic acid, oleic acid and rosin acid.

The quaternary ammonium compounds according to the invention may be prepared by introducing up to 3 methyl groups into the formula of claim 1 with the aid of methyl chloride.

The starting product required for the preparation of the present amines is obtained by reacting an $\alpha$-olefin having 6 to 45 carbon atoms with acetic anhydride at a temperature in the range of 100° to 140°C in the presence of a catalytic amount of an at least trivalent manganese compound. The $\alpha$-olefin may consist of a pure olefin fraction, such as 1-octene, or of a mixture of $\alpha$-olefins having 6 to 45 carbon atoms. If use is made of a mixture of $\alpha$-olefins the number for n in each separate R-radical may, independently of the other R-radicals in the structural formula of the acid and of the amine to be prepared therefrom, assume any value equal to the number of carbon atoms minus two of an $\alpha$-olefin present in the mixture.

The most favourable results are generally obtained at a reaction temperature in the range of 115° to 125°C in the presence of manganic acetate as initiator. To prevent oxidation of the substrate the concentration of the manganic acetate is preferably chosen between $10^{-3}$ and $10^{-10}$ moles per litre.

The concentration of the olefin fraction is dependent on the desired percentage of branched-chain monocarboxylic acids in the reaction product.

If use is made of an olefin fraction having not more than 12 carbon atoms, preference is usually given to a relatively high percentage of branched-chain acids. If, however, use is made of an olefin fraction having 20 to 45 carbon atoms, then there is found to be a strong preference to a mixture of monocarboxylic acids which contains at least 70% by weight of the addition product of 1 mole of olefin to 1 mole of acetic acid. In all cases the reaction conditions will be so chosen that ultimately at least 10% by weight of the branched-chain amines conforms to the first-mentioned structural formula. For the preparation of branched-chain monocarboxylic acids from which the amines according to the above

formula are derived, the molar ratio of converted olefin to manganic acetate is at least 4. It has been found that under these last-mentioned conditions the composition in weight % of the mixture of telomeric acids and, hence, of the amines prepared therefrom is for $n \leqslant 17$ only dependent on the molar ratio of $\alpha$-olefin to manganic acetate and the concentration of the $\alpha$-olefin during the reaction.

With a monocarboxylic acid obtained by reacting one $\alpha$-olefin with acetic acid being indicated by $R_1$, a monocarboxylic acid obtained by reaction with two $\alpha$-olefins by $R_2$, a monocarboxylic acid obtained by reaction with three $\alpha$-olefins by $R_3$, etc., then, for instance, the following weight distributions were obtained respectively before and after removal of the $R_1$-fraction.

| | before distillation wt % | after distillation wt % |
|---|---|---|
| $R_1$ | 30,7 | 0,3 |
| $R_2$ | 20,4 | 19,8 |
| $R_3$ | 21,4 | 33,6 |
| $R_4$ | 13,0 | 21,5 |
| $R_5$ | 9,4 | 15,9 |
| $R \geqslant 6$ | 5,1 | 8,8 |

The structural formulae of $R_3$, $R_4$ and $R_5$ all conform to the first-mentioned formula. $R_1$ is an unbranched acid of the formula $R(CH_2)_3$ COOH and, if $n = 3$ to 9, it is preferably removed from the reaction mixture. The fraction of $R_2$ is formed by two acids of the formula:

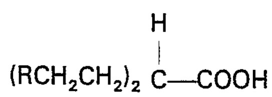

or

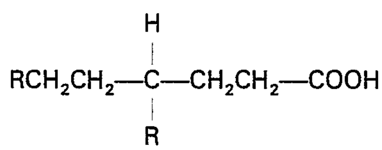

For a man skilled in the art it is obvious that, especially if use is made of an olefin fraction having 30 or more carbon atoms, it is not possible in actual practice to separate the linear acids from the acids having a very high molecular weight and a high degree of telomerization. The same applies to the nitrile and amine derivatives.

The following is a typical example of a weight distribution of the monocarboxylic acids obtained under said conditions and, hence, of the amines prepared therefrom.

| degree of telomerization | wt % |
|---|---|
| m = 1 | 78,0 |
| m = 2 | 6,3 |
| m = 3 | 6,5 |
| m = 4 | 4,0 |
| 3,1 | |
| $m \geqslant 6$ | 2,0 |

It has been found that as far as the above-mentioned field of application of corrosion inhibitors, surface active compounds etc., is concerned, the use of mixtures of amies derived from those branched-chain and straight-chain carboxylic acids lead to compositions having unexpectedly favorable properties, which remarkably favorably compare with the known compositions, which only contain straight-chain amines or the derivatives thereof.

The commercially available olefin fractions having 20 to 45 carbon atoms are found to contain 60 to 80% by weight of $\alpha$-olefins and for the rest predominantly consist of vinylidene compounds.

The resulting amines are $\gamma$-branched monoamines, with the amine having the formula

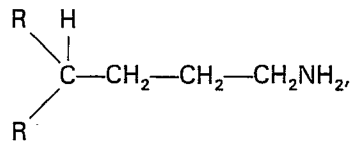

where $R_1$ and $R_2$ represent linear alkyl groups which together have the same number of carbon atoms as the group R.

Separation of these vinylidene groups-containing fractions from the $\alpha$-olefins would give rise to so many technological problems that it must be considered impracticable for economic reasons.

It has been found, however, that for most uses products having exceptionally good properties are obtained if besides the branched-chain amines having the above formulae or the derivatives thereof there is present an amount of 40 to 60 per cent by weight of the amine fraction or of the derivatives thereof which consists of or is derived from linear aliphatic monoamines, with the amine having the formula R $CH_2CH_2CH_2CH_2NH_2$, where R represents a $CH_3(CH_2)n$ group, with n being an integer of from 17 to 42.

The invention is further described in, but not limited by the following examples.

Example I

A slurry made up of 17,2 kg of manganese (III) acetate in 72,6 kg of acetic anhydride and was slowly added, with stirring, over a period of about 6 to 14 hours in an atmosphere of dry nitrogen, to a previously provided mixture of 208 kg (2 kg-moles) of acetic anhydride and 27,2 kg (0,86 kg-moles) of 1-octene. The reaction temperature was 120°C. When the manganese acetate addition was complete, the reaction mixture was cooled and filtered; the reaction product and the filter cake were washed with hexane and the combined liquid phase recharged to the reactor for vacuum stripping. Hydrolysis of the resultant anhydride was accomplished with water/acetic acid, which was finally stripped off under vacuum.

The residual oil contained 38% capric acid, 2% volatiles, and the remainder was telomeric acid.

Fractional vacuum distillation resulted in obtaining three fractions having the following chain-length distribution (given as wt. % according to a GPC analysis) shown in Table I:

TABLE I

| weight % / n* | Fraction A | Fraction B | Fraction C |
|---|---|---|---|
| 1 | 0,1 | 0,3 | 0,7 |
| 2 | 1,3 | 1,0 | 5,0 |
| 3 | 84,9 | 12,1 | 2,4 |
| 4 | 3,9 | 61,9 | 39,5 |
| 5 | 8,3 | 20,0 | 40,6 |
| 6 | 1,4 | 4,7 | 11,8 |

* n = degree of telomerization

The telomeric acid fractions were converted into nitrile batchwise by bubbling ammonia gas through the hot (325°C) acid containing 0,5% catalytic alumina and separating the evolved water via a Dean-Stark trap. The reaction was continued until the conversion to nitrile was approximately 92% (15 to 20 hours). The nitrile was vacuum distilled (yield 85%) and subsequently hydrogenated.

The hydrogenation to primary amine was carried out in a 2,8 cm × 45,7 cm continuous column containing a cobalt catalyst in the form of 0,44 cm extrudates.

Hydrogenation was performed downflow at 120°C (fraction C) and 145°C (fractions A and B), a pressure of 70 x $10^5$ Pa, and a space velocity of 0,7 ml of nitrile 1 hr/ml of bed space.

The molar ratios of $H_2/NH_3$/nitrile were 21:27:1 (fractions A and B) and 15:30:1 (fraction C). Of

the resulting mixture of amines the chemical analysis and the pour point (in conformity with ASTM D97—66) were determined and are shown in Table II.

The weight percentages of primary, secondary, and tertiary amine shown in Table II are based upon the NE values of the parent acids (A=330, B=620, C=1170) and the weight percentages have not been normalized.

TABLE II

| amine \ weight % | Fraction A | B | C |
|---|---|---|---|
| PA | 94 | 106 | 71 |
| SA | 10 | 3 | 9 |
| TA | 3 | 13 | 9 |
| Pour Point | − 50 | − 39 | + 8 |

PA = primary amine, SA = secondary amine, and TA = tertiary amine.

Example II

In this example dialkyl secondary amines were prepared from the primary amines of the preceding example under the following hydrogenation conditions:

| | |
|---|---|
| temperature | 230°—235°C |
| hydrogen pressure | 3,5 × $10^5$ Pa (with vent) |
| powdered Ni-catalyst | 0,5 % by weight |

The reaction time varied from 7 hours (fractions A and B) to 20 hours (fraction C).

Of the resulting products the chemical analysis and the pour point are given in Table III.

TABLE III

| amine \ weight % | Fraction A | B | C |
|---|---|---|---|
| PA | 3 | 6 | 8 |
| SA | 93 | 93 | 89 |
| TA | 4 | 1 | 8 |
| Pour Point | − 50 | − 43 | − 25 |

Example III

Fractions A, B, and C of Example I were cyanoethylated using 10% methanol (based on primary amine) as catalyst at approximately 78°C (reflux). Acrylonitrile was added over thirty minutes and the mixture was refluxed for 2,5 hours. The volatiles were removed in vacuo, and the cyanoamine was reduced to the corresponding diamine under the following reaction conditions:

| | |
|---|---|
| powdered cobalt catalyst | 0,6% by weight (based on primary amine) |
| temperature | 130°C |
| partial pressure | $28.10^5$ Pa $NH_3$ |
| | $28.10^5$ Pa $H_2$ |

The reaction time was 6—12 hours.
The results are given in Table IV:

TABLE IV

| equivalent ratio / amine | Fraction A | Fraction B | Fraction C |
|---|---|---|---|
| PA | 53 | 55 | 62 |
| SA | 47 | 45 | 38 |
| Pour Point | − 45 | − 32 | − 12 |

Example IV

Fractions A, B and C of Example I were ethoxylated to two- and ten-mole ethoxylates under standard conditions.

Two equivalents of ethylene oxide were added from a tared bomb to the amine ($N_2$ purged) at 170°C and 3,5 x $10^5$ Pa. After two hours, the reaction products were cooled and sampled. The ten-mole ethoxylate was prepared from the two mole adduct at 180°C using 1,3% of a 50% aqueous caustic soda solution. The results for the two-mole ethoxylate are shown in Table V. The ten-mole ethoxylate had a pour point of —20°C for fraction A, —14°C for fraction B, and —10°C for fraction C.

TABLE V

| weight % / amount | Fraction A | Fraction B | Fraction C |
|---|---|---|---|
| PA | 1 | 1 | |
| SA | 3 | 5 | |
| TA | 96 | 94 | 100 |
| Pour Point °C | − 28 | − 22 | − 17 |

Example V

Fractions A, B and C of Example I were quaternized with methyl chloride at a pressure of $5,6.10^5$ Pa and a temperature of 80°C, using 2-propanol as the solvent.

Sodium bicarbonate was used to neutralize the two equivalents of HCl liberated by the reaction. The reaction vessel was vented periodically to remove carbon dioxide and repressurized with methyl chloride. The reaction times varied from 4 to 8 hours.

Example VI

In this Example it will be shown that the branched-chain telomeric amines of the present invention are suitable to be used in bitumen adhesion.

The test procedure was as follows:

1 g of the amine was added to 100 g cut-back bitumen and thoroughly stirred to ensure proper dispersion.

A metal container (approximately 12,7 cm in diameter) was covered with 15—20 g of said bitumen to a film thickness of about 1,5 mm.

After cooling to 20°C the container was immersed in water to a depth of about 2,54 cm. Into the bitumen film there were then lightly pressed 10 pieces of granite aggregate having a diameter of about 1,3 cm.

The aggregate was then removed from the binder and the percentage of binder retained on the stone was visually assessed. Both fraction A of Example I and fractions A and B of Example III resulted in 100% coverage.

## Example VII

In this Example it is shown that the branched-chain telomeric amines of the present invention are suitable to be incorporated into acid pickling inhibitor formulations.

The test consists in storing weighed mild steel coupons in inhibited acid for 3 hours at 90°C and subsequently determining the loss in weight. The test procedure was as follows:

The steel coupons were first degreased in boiling carbon tetrachloride for 5—10 minutes and subsequently in boiling acetone for another 5—10 minutes. Subsequently, they were allowed to cool in a desiccator and weighed.

The coupon was then transferred to a bottle containing 200 ml of a hydrochloric acid pickling solution. The hydrochloric acid (15% w/v) contained a 1% solution of the test material. The temperature of the solution was then kept at 90°C for 3 hours, after which the coupons were removed from the acid, washed thoroughly with water and placed in boiling acetone for 5—10 minutes. After cooling they were re-weighed and the loss in weight was calculated.

The results mentioned in the following table clearly show the superiority of the presently claimed amines to the commercially available straight-chain ones.

| Product | Weight loss % by weight |
|---|---|
| Commercial corrosion inhibitor containing a straight-chain amine | 0,25 |
| Same formulation based on an amine of | |
| Example I A | 0,13 |
| Example I C | 0,20 |
| Example III A | 0,23 |
| Example III B | 0,17 |

## Example VIII

The same procedure was used in Example VII, except that the test was carried out now in sulphuric acid (10% by weight). The concentration of the inhibitor formulation was in this case 0,05% by weight.

The loss in weight of the formulation based on a commercial straight-chain amine was 0,40% by weight, whereas the loss in weight of the same formulation based on the amine of Example IV A was as low as 0,33% by weight.

## Example IX

This Example shows the step-wise preparation of a quaternary ammonium compound based on telomeric acid derived from hexene-1.

A. Preparation of Nitrile

650 g of a telomeric acid, derived from hexene-1, having an acid value of 160, a saponification value of 194 and a composition of:

| | |
|---|---|
| 1,6% | m = 1 |
| 16% | m = 2 |
| 29% | m = 3 |

| | |
|---|---|
| 20% | m = 4 |
| 12% | m = 5 |
| 17% | m ⩾ 6 |

and 3,5 g of cobalt (II) oxide were heated in a stainless steel autoclave at 325°C. Dry ammonia gas was continuously fed to the reactor. A pressure of 6 to 8 bar gauge was maintained by careful venting, removing at the same time the reaction water. After 7,5 hours the reaction was stopped. 79,8 g aqueous phase was collected from the vent line together with 36,2 g fatty acid material. 528 g reaction product remained in the autoclave. The reaction product was distilled at a pressure of 40,0 Pa (0.3 Torr) and a top temperature of 170°C, yielding 76,3% w/w distillate and 22,9% w/w residue. The distillate contained 92,1% w/w nitrile, 1,2% w/w telomeric acid and 6,7% w/w apparent soap and amide.

B. Preparation of Primary Amine

120 g of this distillate, being the telomeric nitrile, derived from hexene-1, was heated in a Hastelloy "C" autoclave together with 5,4 g B133 Co-catalyst (Degussa) and 12 g ammonia for 5 hours at 180°—200°C. The autoclave was pressurized with hydrogen to 100 bar gauge. The filtered reaction product contained 74,6% w/w primary amine, 13,1% w/w secondary amine and 12,3% w/w tertiary.

C. Preparation of Quaternary Ammonium Compound

96,2 g of the mixture of amines was heated in a glass autoclave together with 300 g isopropanol, 124,6 g sodium carbonate decahydrate and 109,1 g methyl chloride at 100°C for 5,5 hours, exerting a pressure of 6 bar absolute. At the end of the reaction, the inorganic salt was filtered off and the isopropanol and water were evaporated at 2,7 kPa (20 Torr) and 70°C, yielding 112,2 g reaction product. Analysis of the product revealed that the quaternary ammonium chloride content was 1,86 meq/g, corresponding with approx. 94% w/w. From the composition of the starting amine, it can be calculated that this consists of 73% w/w monotelomeric trimethyl ammonium chloride, 11% ditelomeric dimethyl ammonium chloride and 10% tritelomeric methyl ammonium chloride.

Other analytical data: 0,8% w/w water, 0,1% w/w free amine, 0,1% w/w amine hydrochloric acid salt.

The product appeared to be a liquid at room temperature. This liquidity in solvent-free form is a remarkable advantage over e.g. stearyl trimethyl ammonium chloride which melts only over 200°C and must be diluted with alcohol/water to get lower melting points.

## Example X

This Example shows the step-wise preparation of quaternary ammonium compound based on telomeric acid derived from octene-1.

A. Preparation of Nitrile

682 g of a telomeric acid, derived from octene-1, having an acid value of 168, a saponification value of 187 and a composition of:

| | |
|---|---|
| <1% | m = 1 |
| 87% | m = 2 |
| 11% | m = 3 |
| <1% | m = 4 |

and 4,4 g cobalt (II) oxide were heated in a stainless steel autoclave at 325°C. Dry ammonia gas was continuously fed to the reactor. A pressure of 6 to 8 bar gauge was maintained by careful venting, removing at the same time the reaction water. After 12 hours the reaction was stopped. 95 g aqueous phase was collected from the vent line together with 28,5 g fatty acid material.

566,1 g reaction product remained in the autoclave, containing about 1% w/w telomeric acid and 4,8% w/w apparent soap and amide. The reaction product was distilled at a pressure of 160Pa (1,2 Torr) and a top temperature of 170°C, yielding 84% w/w distillate and 14,4 w/w residue.

The distillate contained 94,9% w/w nitrile, 0,2% w/w telomeric acid and 4,8% w/w apparent soap and amide.

B. Preparation of Secondary Amine

120 g of this distillate, being the telomeric nitrile, derived from octene-1, was heated in a Hastelloy "C" autoclave together with 3,2 g Co-catalyst for 2,5 hours at 180°C (step 1) and for 2,5 hours at 220°C (step 2). During step 1 the autoclave was pressurized with hydrogen to 50 bar gauge.

During both step 1 and 2 the autoclave was periodically vented and flushed with hydrogen. 115,7 g of filtered reaction product was obtained, containing 3,6% w/w primary amine, 87,6 w/w secondary amine and 8,9% w/w tertiary amine.

C. Preparation of Quaternary Ammonium Compound

102,4 g of this mixture of amines was heated in a glass autoclave together with 300 g isopropanol, 53,6 g sodium carbonate decahydrate and 47,6 g methyl chloride at 100°C for 6 hours, exerting a pressure of 6 bar absolute. At the end of the reaction the salts were filtered off and the isopropanol and water were distilled off at 2,7 kPa (20 Torr) and 70°C, yielding 111,4 g reaction product.

Analysis of the product revealed that the quaternary ammonium chloride content was 1,51 meq/g, corresponding with approximately 92% w/w. From the composition of the starting amine it can be calculated that this consists of 4% w/w monotelomeric trimethyl ammonium chloride, 80% w/w ditelomeric dimethyl ammonium chloride and 8% w/w tritelomeric methyl ammonium chloride.

Other analytical data: 0,1% w/w free amine, 0,2% w/w amine hydrochloric acid salt and 0,1% water.

The product appeared to be liquid at room temperature. This liquidity is a remarkable advantage over distearyl dimethyl ammonium chloride, which melts at 115°C in pure form and at approximately 40°C when diluted with 25% isopropanol/water.

Example XI

This Example demonstrates the ability of the two quaternary ammonium compounds from Examples IX and X to demulsify oil-in-water emulsions.

The quaternary ammonium compounds of the Examples are compared to a commercially available nonionic surfactant of the structure

$HO(EO)_a(PO)_b(EO)_cH$, wherein EO represents ethylene oxide linkages and PO represents propylene linkages, sold under the trademark Dissolvan 4460 by Hoechst.

Standard emulsions were prepared by mixing equal amounts (50 ml oil/50 ml water) of oil from the Forties field in the North Sea and water in an Ultra Turrax mixer for 20 minutes at maximum speed. The temperature of the emulsions during mixing was maintained at a maximum of 40°C through ice cooling.

Immediately after the emulsions were prepared, they were conditioned at 40°C and 60°C in 100 ml calibrated glass cylinders. Subsequently, the demulsifier to be tested was added in isopropanol at concentrations of 10, 25, 50 and 100 ppm, and the mixture homogenized by shaking manually. The amount of water removed was measured as a function of time and the demulsifications were performed at both 40°C and 60°C. The results of the demulsifications are shown in the following Tables VI and VII and demonstrate that the quaternary ammonium compound of Examples XI and X both demulsify the oil/water emulsion with the compound of Example IX being more effective than the compound of Example X, but less effective than the commercially available demulsifier Dissolvan 4460. The corresponding dimethyldicocoammonium chloride did not act as demulsifier.

## TABLE VI

Demulsification of Forties emulsions (50 ml oil, 50 ml water)
T = 40°C. Figures denote ml of water separated

| Demulsifier/Conc. ppm | 10 min. | 20 min. | 30 min. | 1 h | 2 h | 3 h | 4 h | 5 h | 24 h |
|---|---|---|---|---|---|---|---|---|---|
| Example IX / 10 ppm | | | | | | | 1 | 30 | |
| Example IX / 25 ppm | | | | | 2 | | 43 | 45 | |
| Example IX / 50 ppm | | | 39 | 45 | 48 | 48 | 49 | 49 | |
| Example IX / 100 ppm | | 43 | 44 | 45 | 48 | 49 | 49 | 49 | |
| Example X / 10 ppm | | | | | | | 1 | 1 | |
| Example X / 25 ppm | | | | | | | 21 | 36 | |
| Example X / 50 ppm | | | 1 | 4 | 27 | 42 | 42 | 45 | |
| Example X / 100 ppm | | 14 | 47 | 49 | 49 | 49 | 49 | 49 | |
| Dissolvan 4460/10 ppm | | | | | 4 | | | | 48 |
| Dissolvan 4460/50 ppm | 43 | 46 | 48 | 48 | | | | | |
| Dissolvan 4460/100 ppm | 49 | | | | | | | | |

## TABLE VII

Demulsification of Forties emulsions (50 ml oil, 50 ml water)
T = 60°C. Figures denote ml of water separated

| Demulsifier/Conc. ppm | 2 min. | 8 min. | 15 min. | 30 min. | 1 h | 2 h | 4 h | 24 h |
|---|---|---|---|---|---|---|---|---|
| Example IX / 10 ppm | | | | 5 | 33 | 48 | 50 | |
| Example IX / 25 ppm | | | 5 | 43 | 49 | 50 | | |
| Example IX / 50 ppm | | 44 | 46 | 47 | 50 | | | |
| Example IX / 100 ppm | 22 | 46 | 48 | 49 | | | | |
| Example X / 10 ppm | | | | | 8 | 44 | 47 | 49 |
| Example X / 25 ppm | | | | 24 | 44 | 50 | | |
| Example X / 50 ppm | | 1 | 3 | 42 | 50 | | | |
| Example X / 100 ppm | 1 | 32 | 50 | | | | | |

Example XII

This Example shows the step-wise preparation of a diamine based on telomeric acid derived from 1-decene.

A. Preparation of Nitrile

A telomer acid derived from 1-decene, acetic anhydride, and manganese III acetate utilizing a process similar to Example I, having a neutralization equivalent of 514 was converted to nitrile batchwise by bubbling ammonia gas through hot (325°C) acid containing 0,5% catalytic alumina and separating the evolved water via a Dean-Stark trap. After 10 hours the conversion to nitrile was approximately 93%.

B. Preparation of Monoamine

This nitrile from A above, without separation, was reduced utilizing a continuous downflow process employing a cobalt catalyst. The reaction temperature was 140°C, the pressure was $70 \times 10^5$ Pa, the space velocity was 0,7, and the molar ratio of $H_2/NH_3$/nitrile was 12:17:1. The resulting amine analyzed as being 80% primary amine, 7% secondary amine, 5% tertiary amine (based on theoretical molecular weight from acid).

C. Preparation of Diamine

The amine from B was cyanoethylated using 8% methanol catalyst (based on amine). Acrylonitrile was added over 25 minutes and the mixture was refluxed for 9 hours. The volatiles were removed in vacuo, and the cyanoamine was reduced to the corresponding diamine under the following condition:

| | |
|---|---|
| catalyst (Raney Nickel) | 1% by weight |
| temperature | 130°C |
| pressure | $28 \times 10^5$ Pa $NH_3$ |
| | $28 \times 10^5$ Pa $H_2$ |

The reduction time was 4 hours and the resultant diamine content was 70%

**Claims**

1. A mixture of amines, at least part of which are branched-chain amines, or the secondary, tertiary or quaternary amines derived therefrom and containing 1 up to 3 N-substituted groups selected from the group consisting of substituted or unsubstituted alkyl groups having up to 40 carbon atoms, substituted or unsubstituted cycloalkyl groups having from 3 to 6 carbon atoms and/or polyalkylene oxide-containing groups having from 2 to 30 alkylene oxide units each containing from 2 to 4 carbon atoms, characterized in that at least 10% by weight of the branched-chain amines conforms to the formula:

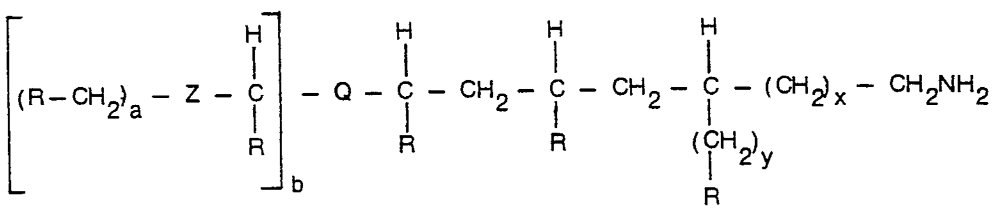

where x is 0 or 2 and where y is 2 or 0, and where
when y = 0, x = 2 and
when y = 2, x = 0;
R = $CH_3(CH_2)_n$, where n represents an integer of from 3 to 42
b = 0 or 1, where
when b = 0, Q represents a hydrogen atom, and
when b = 1, Q represents a $CH_2$-group, and
a = 0 or 1, where
when a = 0, Z represents a hydrogen atom, and
when a = 1, Z represents a $CH_2$-group.

2. A mixture of amines or the secondary, tertiary or quaternary amines derived therefrom according to claim 1, characterized in that n represents an integer of from 3 to 17.

3. A mixture of amines or the secondary, tertiary or quaternary amines derived therefrom

according to claim 1, characterized in that n represents an integer of from 17 to 42.

4. A mixture of the secondary, tertiary or quaternary amines according to claim 1, characterized in that the alkyl groups or the cycloalkyl groups are substituted with one or more lower alkyl or lower alkoxy groups, an amino group, a hydroxyl group, a halogen atom, a cyano group, or a nitro group.

**Patentansprüche**

1. Eine Mischung von Aminen, von denen mindestens ein Anteil verzweigtkettige Amine sind, oder davon abgeleitete sekundäre, tertiäre oder quaternäre Amine und welche 1 bis 3 N-substituierte Gruppen enthalten, ausgewählt aus der Gruppe bestehend aus substituierten oder nicht substituierten Alkylgruppen mit bis zu 40 Kohlenstoffatomen, substituierten oder nicht substituierten Cycloalkylgruppen mit 3 bis 6 Kohlenstoffatomen und/oder Polyalkylenoxid enthaltende Gruppen mit 2 bis 30 Alkylenoxideinheiten, welche jeweils 2 bis 4 Kohlenstoffatomen enthalten, dadurch gekennzeichnet, daß mindestens 10 Gewichtsprozent der verzweigtkettigen Amine der nachstehenden Formel entsprechen:

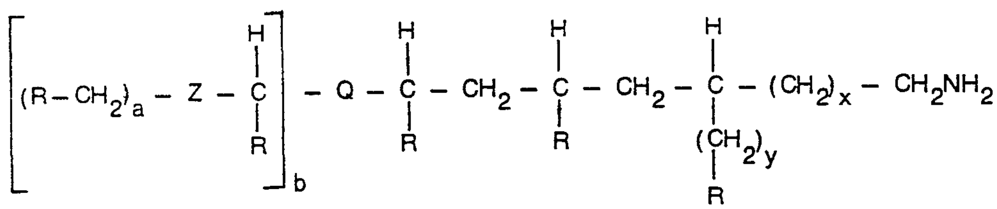

in welcher x 0 oder 2, und in welcher y oder 2 oder 0 ist, und wobei

falls y=0, x=2 und

falls y=2, x=0;

$R=CH_3(CH_2)_n$ ist, wobei n eine ganze Zahl von 3 bis 42 bedeutet,

b=0 oder 1 ist, wobei

falls b = 0, Q ein Wasserstoffatom bedeutet, und

falls b = 1, Q eine $CH_2$-Gruppe bedeutet,

und a = 0 oder 1 ist, wobei

falls a = 0, Z ein Wasserstoffatom bedeutet, und

falls a = 1, Z eine $CH_2$-Gruppe bedeutet.

2. Eine Mischung von Aminen oder davon abgeleiteten sekundären, tertiären oder quaternären Aminen gemäß Anspruch 1, dadurch gekennzeichnet, daß n eine ganze Zahl von 3 bis 17 ist.

3. Eine Mischung von Aminen oder davon abgeleiteten, sekundären, tertiären oder quaternären Aminen gemäß Anspruch 1, dadurch gekennzeichnet, daß n eine ganze Zahl von 17 bis 42 ist.

4. Eine Mischung von sekundären, tertiären oder quaternären Aminen gemäß Anspruch 1, dadurch gekennzeichnet, daß die Alkylgruppen oder die Cycloalkylgruppen mit einer oder mehreren Niederalkyl- oder Niederalkoxygruppen, einer Aminogruppe, einer Hydroxylgruppe, einem Halogenatom, einer Cyanogruppe oder einer Nitrogruppe substituiert sind.

**Revendications**

1. Un mélange d'amines qui sont au moins en partie des amines ramifiées, ou les amines secondaires, tertiaires ou quaternaires en dérivant et contenant 1 à 3 groupes N-substitués choisis parmi le groupe constitué par les groupes alkyles substitués ou non substitués ayant jusqu'à 40 atomes de carbone, les groupes cycloalkyles substitués ou non substitués ayant 3 à 6 atomes de carbone et/ou les groupes contenant un polyoxyde d'alkylène ayant 2 à 30 motifs d'oxyde d'alkylène contenant chacun 2 à 4 atomes de carbone, caractérisé en ce qu'au moins 10% en poids des amines à chaîne ramifiée répondent à la formule:

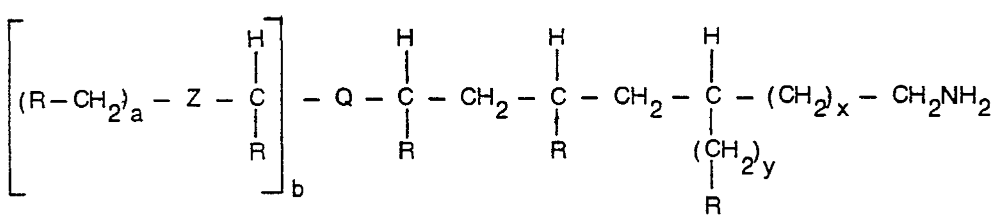

où x est 0 ou 2 et où y est 2 ou 0,

et où lorsque $y = 0$, $x = 2$, et
lorsque $y = 2$, $x = 0$;
$R = CH_3(CH_2)_n$, où n représente un entier de 3 à 42,
$b = 0$ ou 1, où
lorsque $b = 0$, Q représente un atome d'hydrogène, et
lorsque $b = 1$, Q représente un groupe $CH_2$, et
$a = 0$ ou 1, où
lorsque $a = 0$, Z représente un atome d'hydrogène, et
lorsque $a = 1$, Z représente un groupe $CH_2$.

2. Un mélange d'amines ou les amines secondaires, tertiaires ou quaternaires en dérivant selon la revendication 1, caractérisé en ce que n est un entier de 3 à 17.

3. Un mélange d'amines ou les amines secondaires, tertiaires ou quaternaires en dérivant selon la revendication 1, caractérisé en ce que n est un entier de 17 à 42.

4. Un mélange d'amines secondaires, tertiaires ou quaternaires selon la revendication 1, caractérisé en ce que les groupes alkyles ou les groupes cycloalkyles sont substitués par un ou plusieurs groupes alkyles inférieurs ou alcoxy inférieurs, un groupe amino, un groupe hydroxy, un atome d'halogène, un groupe cyano ou un groupe nitro.